Europäisches Patentamt

European Patent Office

Office européen des brevets

(19)

(11) **EP 1 595 958 A1**

(12) **EUROPEAN PATENT APPLICATION**

(43) Date of publication:
**16.11.2005 Bulletin 2005/46**

(51) Int Cl.7: **C12Q 1/68**

(21) Application number: **04076394.8**

(22) Date of filing: **10.05.2004**

| | |
|---|---|
| (84) Designated Contracting States:<br>**AT BE BG CH CY CZ DE DK EE ES FI FR GB GR HU IE IT LI LU MC NL PL PT RO SE SI SK TR**<br>Designated Extension States:<br>**AL HR LT LV MK**<br><br>(71) Applicants:<br>• **Nederlandse Organisatie voor Toegepast-Natuurwetenschappelijk Onderzoek TNO**<br>**2628 VK Delft (NL)**<br>• **UMC Utrecht Holding B.V.**<br>**3584 CM Utrecht (NL)** | (72) Inventors:<br>• **Schuren, Frank Henri Johan**<br>**3902 EB Veenendaal (NL)**<br>• **Montijn, Roy Christiaan**<br>**1024 NL Amsterdam (NL)**<br>• **Verhoef, Jan**<br>**3707 EM Zeist (NL)**<br><br>(74) Representative:<br>**Winckels, Johannes Hubertus F. et al**<br>**Vereenigde**<br>**Johan de Wittlaan 7**<br>**2517 JR Den Haag (NL)** |

(54) **Staphylococcus aureus specific array and diagnostics**

(57)    Means and methods are provided for typing sample nucleic acid derived from *Staphylococcus aureus*. Typing is performed using an array of nucleic acids wherein the detector nucleic acid comprises a size of between about 200 and 5000 nucleotides and wherein the sample nucleic acid comprises an average size of between 50 and 5000 nucleotides. Hybridization is compared with a reference pattern. There is no need to specifically type the detector nucleic acid.

**EP 1 595 958 A1**

Printed by Jouve, 75001 PARIS (FR)

**Description**

The invention relates to the fields of diagnostics for *Staphylococcus aureus* and arrays therefore.

*Staphylococcus aureus* is a major problem in the care of hospitalized patients. There are many different *Staphylococcus aureus* strains, some of which are resistant to a wide spectrum of antibiotics. The different strains of *Staphylococcus aureus* behave differently with respect to their infectiousness. Some strains rapidly spread from patient to patient, whereas other strains do not spread that easily. Considering that at least some of the *Staphylococcus aureus* strains are capable of causing severe disease in hospitalized patients, hospitals maintain very strict hygiene rules. It has previously not been possible to quickly determine whether a particular strain is a fast spreading strain (epidemic strain) or not. A hospital that is faced with a methicillin resistant *Staphylococcus aureus* (MRSA) infected patient thus has no choice but to enforce the most stringent quarantine and hygienic rules. In one aspect the present invention provides means and methods with which it is possible to quickly determine whether an MRSA strain is epidemic or not. The invention is also suited to determine other characteristics of particular *Staphylococcus aureus* strains. To this end the invention provides specific arrays that are capable of distinguishing between the various strains of *Staphylococcus aureus* and more importantly, to estimate properties of a particular *Staphylococcus aureus* strain, even under circumstances wherein patterns generated on the array are not identical to an already earlier generated pattern.

Arrays of the invention contain at least 500.000 nucleotides on them. Preferably the arrays carry even more nucleotides on them. In a preferred embodiment the array comprises at least 1 megabase ($10^6$ nucleotides). Preferably, they comprise at least 2 megabases. The arrays preferably carry at least 70 % of a single *Staphylococcus aureus* genome, or at least 70% of the coding regions of *Staphylococcus aureus.* Contrary to conventional arrays, the number of bases per DNA molecule in a spot is high, i.e. between 50 and 5000 nucleotides. In conventional arrays, the number of nucleotides per DNA molecule in a spot is kept low for reasons of economy and precision. Generating larger oligonucleotides increases the price of the oligonucleotide and simultaneously introduces a larger propensity for errors with respect to the desired sequence. In the present invention, this drawback is, at least in part, overcome through the preferred use of nucleic acid that is obtained or derived from a natural source, preferably living material. The introduction of larger numbers of nucleotides in the array also introduces another difficulty, particularly when complex nucleic acid is used to probe the array. In this situation a larger number of spots have signals between the value 0 and 1, indicative for the fact that not all of the nucleic acid in the spot is hybridized to probe nucleic acid. Although arrays of the invention can be used to identify specific sequences in the probe nucleic acid, and thus to type the probe nucleic acid on the basis of a signal in one or a few spots, the full potential of the array lies in the interpretation of signals obtained in many spots. This interpretation can be done by a human but is typically done by a computer using statistical software.

Thus in one aspect the invention provides a method for typing sample nucleic acid derived or obtained from a *Staphylococcus aureus* strain comprising, providing an array comprising a plurality of nucleic acid molecules obtained or derived from *Staphylococcus aureus* wherein said array nucleic acid molecules comprise an average size of between about 200 to 5000 nucleotides, said method further comprising hybridizing said array with *Staphylococcus aureus* sample nucleic acid and comparing the sample hybridization pattern with a reference pattern. The average size of sample nucleic acid has an effect on the signal distribution on the array. Larger sample molecules comprise more information and are thus more likely to find a suitable hybridization partner in more of the spots. Reducing the average size of the sample nucleic acid can reduce this phenomenon. On the other hand, when the sample nucleic acid is too small, the nucleic acid fragments in the sample contain too little genetic information and also find suitable hybridization partners in many spots. The average size of the fragments in the sample nucleic acid is preferably between about 50 and 5000 nucleotides. More preferably, the average size of the fragments in the sample nucleic acid comprises a size of between about 50 and 1000 nucleotides, more preferably between about 50 and 500 nucleotides.

The sample nucleic acid preferably represents the whole sample genome. The pattern obtained with the sample nucleic acid on the array is compared with a reference pattern. The reference pattern can be artificially generated, for instance, through using knowledge of the nucleic acid composition of a reference sample, for instance the genome sequence of a *Staphylococcus aureus* strain of which the genomic sequence is known. However, in a preferred embodiment, the reference pattern is generated by hybridizing reference nucleic acid to the array. Comparison of the sample pattern with the reference can at least be used to determine whether the sample nucleic acid is the same or similar to the reference nucleic acid. This is useful when one needs to determine whether, for instance, the sample nucleic acid contains a particular *Staphylococcus aureus* strain. In this setting a reference pattern is generated with nucleic acid of the particular *Staphylococcus aureus* strain and when the sample pattern is essentially the same as the reference pattern, the sample is identified as containing the particular *Staphylococcus aureus* strain. In a preferred embodiment a method of the invention further comprises comparing the sample pattern with at least one other reference pattern. In this way the sample can be compared to at least two different reference nucleic acids. Of course, upon continued use of the array, more and more *Staphylococcus aureus* patterns are generated and all of these can be used to compare with the sample nucleic acid. Thus once a pattern is generated with sample nucleic acid this pattern can in a subsequent experiment be used as a reference pattern. Thus in a preferred embodiment a method of the invention further comprises comparing the hybridization pattern with at least 2, preferably at least 5 and more preferably at least 50 reference patterns. More preferably at least 100, more preferably at least 1000 reference patterns.

The sample pattern and the reference pattern can be a subset of signals obtained from the array. A pattern can consist of one signal; preferably the pattern consists of at least 20 % of the signals obtained following hybridization to the array. More preferably, the pattern consists of at least 50% of the signals from the array. In a particularly preferred embodiment the pattern comprises at least 80% of the signals of the array.

A method of the invention cannot only be used to determine whether a *Staphylococcus aureus* sample nucleic acid is the same as a particular *Staphylococcus aureus* reference nucleic acid. The sample pattern can, as it happens, be different from any of the reference patterns. A particularly useful characteristic of the methods and arrays of the invention is that also in this case a method of the invention can provide useful information. Phenotypic characteristics associated with the *Staphylococcus aureus* strain from which the sample nucleic acid is derived or obtained are very often the result of the interplay of a large number of different sequences and/or genes. In these situations it is not possible to type a particular sample on the basis of the signal obtained in one or more spots. Rather, the signals of very many different spots need to be compared. The methods and arrays of the invention are particularly suited for this type of analysis. To this end the reference patterns and the sample pattern are analyzed using statistical software. In one embodiment, a method of the invention further comprises unsupervised multivariate analysis (e.g. Principal Component Analysis, PCA) of the *Staphylococcus aureus* reference patterns together with the pattern generated by the *Staphylococcus aureus* sample nucleic acid. Based on this analysis a pattern is given an n dimensional value (with n representing a value between 2 and the total number of datapoints included in the analysis), which can be reduced to its preferably 2 principal components. These components can be visualized in a multi-dimensional visualization, preferably in a two-dimensional visualization. The dimensional value of the components can be plotted for all patterns, which are included in the analysis whereupon the grouping or clustering of the preferably two-dimensional values of the patterns can be scrutinized. In a preferred embodiment the two-dimensional value of the sample pattern is compared with al two-dimensional values of the reference patterns. In this way it is possible to provide a statistical estimation of the relatedness of the *Staphylococcus aureus* strain that the sample nucleic acid is derived or obtained from compared tot the included references.

In a preferred embodiment the two-dimensional values of the reference patterns are clustered. This clustering is preferably done on the basis of the relatedness. This typing is typically associated with a margin of error for the classification, i.e. the chance that the sample nucleic acid is wrongly classified as being related to a particular (cluster) of reference strains. Thus a method of the invention preferably further comprises typing the relatedness of a *Staphylococcus aureus* used to generate said sample nucleic acid, on the basis of the presence or absence in a cluster.

The term "clustering" refers to the activity of collecting, assembling or uniting into a cluster or clusters items with the same or similar characteristics, a "cluster" referring to a group or number of the same or similar items gathered or occurring closely together. "Clustered" indicates that an item has been subjected to clustering. The process of clustering used in a method of the present invention may be done by hand or by eye or by any (mathematical) process known to compare items for similarity in characteristics, attributes, properties, qualities, effects, etc., through data from measurable parameters. Statistical analysis may be used.

Principal component analysis (PCA) can be performed with mean centering as the selected scaling method. Comparable results can be obtained using other scaling methods. In a preferred embodiment the mean centering scaling method is used. For a review of Principal component analysis reference is made to Joliffe IT. 1986. Principal Component Analysis. New York: Springer-Verlag.

In another embodiment of the invention patterns are extended with further information on the *Staphylococcus aureus* strain wherefrom a reference and/or sample nucleic acid is obtained or derived. For instance, patterns may be extended with parameters that are determined in a way different from nucleic acid hybridization. For *Staphylococcus aureus* strains it is often important to know the antibiotic resistance phenotype of the strains. This resistance parameter can be added to the statistical analysis. The value of this parameter (resistant or sensitive, or further fine tuning) can be added to the pattern or to the statistical analysis of the patterns. The clustering can subsequently be based on this additional parameter. Thus in a preferred embodiment of the invention, at least one non nucleic acid based parameter is determined for the organisms that were the source of the reference patterns. The statistical analysis can subsequently be used to determine or estimate the value of this parameter for the *Staphylococcus aureus* that was the source of the sample nucleic acid. In this embodiment, a method of the invention further comprises Partial Least Square-Discriminant Analysis (PLS-DA) of the reference patterns together with the pattern generated by the sample nucleic acid, wherein at least one parameter of which the values are known for the reference patterns is used to supervise the PLS-DA analysis.

Partial Least Squares (PLS)

Partial least squares (PLS) has been described extensively in the literature (P. Geladi and B.R. Kowalski, Partial Least Squares Regression: A Tutorial, *Analytica Chimica Acta,* 185, 1986, 1-17. H. Martens and T. Naes, Multivariate Calibration, John Wiley & Sons, Chichester, 1989.) Whereas a principal component analysis (PCA) model has a *descriptive* nature, a PLS model has a *predictive* nature. In PLS, scores*loadings pairs, also called latent variables (LVs), are not calculated to maximise the explained variance in the *predicting data set* only, but also to maximise the covariance with the *data to be predicted.* The PLS model can be summarised mathematically by means of Equation (1) and Equation (2).

$$X = TP^T + E \tag{1}$$

$$Y = TBQ^T + F \tag{2}$$

Matrix **X** (also called X-block) represents a $n * p$ matrix of independent variables ($n$ chromatograms, for example, with $p$ retention times per chromatogram), **Y** (also called Y-block) is a $n * q$ matrix containing the dependent variables (concentrations, for example); $\mathbf{P}^T$ and $\mathbf{Q}^T$ are transpose $S * p$ and $S * q$ matrices, containing the dependent and independent variable loadings, respectively; **T** is an $n * S$ matrix of S latent scores, **B** is a $S * S$ matrix representing the regression of the scores of the **X** matrix on the scores of the **Y**-data; **E** and **F** are $n * p$ and $n * q$ matrices containing the residuals of the independent and dependent variables, respectively.

The standard error of validation (SEV) after extracting A LVs, is calculated from Equation (3).

$$\mathrm{SEV} = \sqrt{\frac{\sum_{I=1}^{I_c} \left(Y_{i,j} - y_{i,j}\right)^2}{I_c}} \tag{3}$$

where $I_c$ is the number of calibration samples, $y_{i,j}$ is the true value for the concentration of component $j$ in object $i$; $Y_{i,j}$ is the PLS predicted value for $y_{ij}$; $q$ is the number of $Y$-variables. The extraction of LVs is continued as long as the SEV is improved significantly.

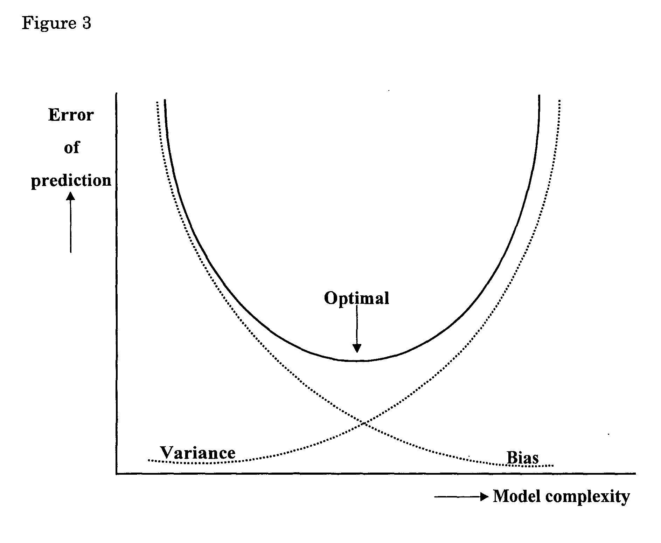

The number of LVs chosen must yield an optimal prediction of the variable of interest. However, there is a pay-off between variance and bias (or fit): a too complex model fits well, but may predict very poorly. This leads to the concept of optimal model complexity: an optimal balance between fit and variance is obtained. This is illustrated graphically in Figure 3, where the increased complexity of the model is able to fit more features in the data, but the variance of the estimated parameters rises and the overall result attains a minimum value at the optimal model complexity.

Pure linear relations between **X** and **Y** will result in a simple model with usually two to five LVs. Complex non-linear relations can also be modelled. However, these will take up significantly more LVs to correlate **Y** to **X**.

**Partial Least Squares - Discriminant Analysis (PLS-DA)**

In PLS-DA classes (predefined groups) are used as dependent variables. The Y-block **Y** is a matrix of n*number of classes. The Y-block is filled with zeros and ones.

Example:

*class* = [12 21]

$$Y = \begin{bmatrix} 1 & 0 \\ 0 & 1 \\ 0 & 1 \\ 1 & 0 \end{bmatrix}$$

Using a Y-block in PLS which is filled with zeros and ones dependent on the class each sample belongs to, turns PLS into a discriminant analysis.

Principal Component-Discriminant Analysis (PC-DA)

Discriminant analysis (DA) [D.L. Massart, B.G.M. Vandeginste, L.M.C. Buydens, S. De Jong, P.J. Lewi and J. Smeyers-Verbeke, Handbook of Chemometrics and Qualimetrics: Part A, Elsevier, Amsterdam, 1997; B.G.M. Vandeginste, D.L. Massart, L.M.C. Buydens, S. De Jong, P.J. Lewi and J. Smeyers-Verbeke, Handbook of Chemometrics and Qualimetrics: Part B, Elsevier, Amsterdam, 1998] is applied if the interest is focused on differences between groups of samples. The technique is based on the assumption that samples of the same group are more similar compared to samples of other groups. The goal of DA is to find and identify structures in the original data, which show large differences in the group means. This process involves a priori knowledge of which samples are similar. Therefore, DA is said to be a *supervised* analysis technique. This distinguishes it from other *unsupervised* techniques such as principal component analysis (PCA), for example, which does not require a priori knowledge about samples.

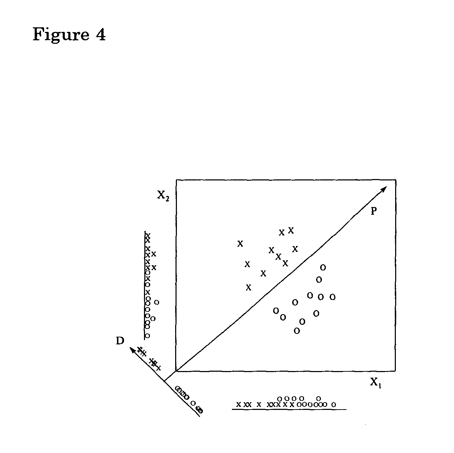

The first step in DA is to combine the original variables into a set of mutually independent new variables in such a way that the projection of the original samples in the space, spanned by a minimum number of these new variables, maximises the difference between the group means. This principle is demonstrated in Figure 4. Two groups of samples are measured on two variables $X_1$ and $X_2$. Using the principal component (PC) maximum variance criterion these samples should be projected on the line through the samples as indicated in Figure 4 by line P. For discriminating between the different clusters of samples this is not the optimal solution. However, the projection of the samples on line D shows a complete separation between the two clusters. The calculated factors are called discriminants or D-axes. All other projections give sub-optimal solutions. This is demonstrated in the figure 4 by comparing the projection of the samples on de D-line with those on the $X_1$ or $X_2$ axis.

DA describes most efficiently the differences between groups of samples. However, the number of variables is often large compared to the number of samples. This may lead to degenerate solutions. For instance three samples can always be separated by two variables independent of their similarity. If more samples are included this degeneracy effect will disappear. The general rule of thumb is that the number of samples should be at least four times the number of variables. This rule can lead to problems in the examination of nuclear magnetic resonance (NMR) spectra, for example. In the analysis of natural products the number of peaks (variables) per NMR spectrum is generally in the order of a few hundred. Under normal circumstances this would mean that one should measure at least 400 to 800 samples. In practice this never occurs. Based on this it would be impossible to perform DA on NMR spectra of natural products. However, there is a solution to this problem. Hoogerbrugge *et al.* [R. Hoogerbrugge, S.J. Willig and P.G. Kistemaker, Discriminant Analysis by Double Stage Principal Component Analysis, *Analytical Chemistry,* 55, 1983, 1710-1712.] developed a scheme in which the number of variables is reduced by PCA, firstly, followed by DA on the scores of the samples on the first PC axes. This technique is called principal component-discriminant analysis (PC-DA). Determining the exact number of PCs to include is difficult. The number should not be too small because including only the first few can result in a loss of a lot of the between-group information. The number should not be too large also, because it will exceed the number-of-samples-divided-by-four rule. Therefore, it seems advisable to include all PCs, which explain a significant amount of variance (for instance above 1% of the original variance) up to a maximum of the number of samples divided by four.

If the total amount of variance explained by these PCs is very low then the number can always be increased. However, if the explained variance is low, the correlations between the original variables will be low also. As a consequence DA will generate a result which will be as complicated as the original problem.

The parameter used in the PLS-DA analysis is preferably a phenotypic parameter. The term "phenotypic parameter is used here to define any parameter that describes any property of that is exhibited or expressed by the *Staphylococcus aureus* or a functional part thereof. Based on this analysis a pattern is given a n dimensional value (with n representing a value between 2 and the total number of discriminating datapoints included in the analysis), which can be reduced to its, preferably two, principal components for optimal correlation with the phenotypic parameter in a, preferably, two dimensional visualization. This preferably two-dimensional value can be plotted for all patterns whereupon the grouping or clustering of the preferably two-dimensional values of the patterns can be scrutinized. In a preferred embodiment the two-dimensional value of the sample pattern is compared with all two-dimensional values of the reference patterns. In this way it is possible to provide a statistical estimation of the chance that the *Staphylococcus aureus* strain that the sample nucleic acid is obtained or derived from comprises a certain phenotypic characteristic or not. This of course necessitates that this phenotypic characteristic is known for the *Staphylococcus aureus* strains from which the reference nucleic acid is obtained or derived. In a preferred embodiment the two-dimensional values of the reference patterns are clustered based on the supervised PLS-DA analysis. This clustering is preferably done on the basis of the phenotypic characteristic for which the sample pattern is scrutinized. This clustering preferably results in two clusters, wherein one cluster has the certain phenotype and the other has not. The sample pattern can thus easily be identified as having or not having the certain phenotype. This typing is typically associated with a margin of error for the classification, i.e. the chance that the sample nucleic acid is wrongly classified as having or not having the certain phenotypic characteristic. The borders of the clusters can be set to accommodate a smaller or larger statistical chance of error. Thus a method of the invention preferably further comprises typing said sample nucleic acid on the basis of the presence or absence in a cluster. Preferably, a method of the invention, further comprising typing said sample nucleic acid on the basis of the presence or absence in a cluster. In a preferred embodiment the parameter comprises epidimicity. Preferably, the patterns are clustered or grouped on the basis of their epidemic phenotype, i.e. the potential for spread of the strain to other patients in a hospital setting.

The reference pattern can be generated from a wide variety of nucleic acids. As mentioned above, the reference pattern is preferably generated from a nucleic acid obtained or derived from a natural *Staphylococcus aureus* source. Preferably, at least 5 and more preferably at least 50 reference patterns are generated by nucleic acid obtained or derived from different *Staphylococcus aureus* strains. In a particularly preferred embodiment essentially all reference patterns are generated by nucleic acid of *Staphylococcus aureus* strains. In this way it is possible to type a sample for the presence therein of nucleic acid derived or comprises a similar phenotypic parameter as a certain strain or strains of *Staphylococcus aureus.* This particularly preferred embodiment is preferably combined with a statistical analysis of the reference and sample pattern. In this way it is possible to determine the chance that a *Staphylococcus aureus* in a sample comprises a certain phenotypic characteristic that is shared by some but not all *Staphylococcus aureus* strains. The *Staphylococcus aureus* nucleic acid can be derived from RNA but is preferably derived or obtained from *Staphylococcus aureus* DNA. i.e. derived from the genome. Thus in a preferred embodiment of the invention said plurality of nucleic acid molecules is derived from *Staphylococcus aureus* DNA.

The sample and/or reference nucleic acid used to generate the patterns may contain a subset of nucleic acid of the *Staphylococcus aureus* strain it is derived or obtained from. However, preferably no selections are performed. In any case, selections are preferably the same or similar for sample and reference nucleic acid. This allows for an easy comparison of the reference and the sample patterns.

With the term "nucleic acid obtained or derived from" it is meant that it is not essential that the nucleic acid used to hybridize on the array is directly obtained from the *Staphylococcus aureus* source. It may have undergone cloning, selections and other manipulations prior to the use for hybridizations. Sample and reference nucleic acid can for instance be obtained from cloned libraries, such as expression or genome libraries. Alternatively, sample and reference nucleic acid can be generated from scratch based on the nucleic acid information in databases, for instance, as a result of the ongoing genomics efforts. However, preferably sample and reference nucleic acid are obtained directly, or through amplification from a natural *Staphylococcus aureus* source. Preferably, the sample pattern is generated starting from a monoculture of a *Staphylococcus aureus* strain. In this way it is warranted that only one *Staphylococcus aureus* is analyzed on the array, and in the same time the pattern generated is a pattern generated from one *Staphylococcus aureus* strain. In one aspect, the invention provides an array comprising a plurality of *Staphylococcus aureus* nucleic acid molecules wherein said nucleic acid molecules comprise an average size of between about 200 to 5000 nucleotides. An array of the invention preferably comprises at least 500.000 nucleotides on them. Preferably the arrays carry even more nucleotides on them. In a preferred embodiment the array comprises at least 1 megabase ($10^6$ nucleotides). Preferably, they comprise at least 2 megabases. Contrary to conventional arrays, the number of bases per spot is high, i.e. between 200 and 5000 nucleotides. Preferably, said plurality of nucleic acid molecules are derived from a natural *Staphylococcus aureus* source. Preferably, wherein said plurality of nucleic acid molecules is derived from *Staphylococcus aureus* DNA. It has been found that different strains of *Staphylococcus aureus,* while belonging to the same species can nevertheless vary greatly in the amount and kind of DNA that they carry. Thus in a preferred embodiment, an array of the invention comprises a plurality of nucleic acid molecules that is derived from at least two different strains of *Staphylococcus aureus.* Preferably, the plurality of nucleic acid molecules in the array comprises at least a representation of the genome of a *Staphylococcus aureus* strain. This is preferably extended with nucleic acid derived from at least one other strain of *Staphylococcus aureus.* In this way the array is a more representative of the entire genetic diversity of the *Staphylococcus aureus* species. In a particularly preferred embodiment, the array comprises a plurality of nucleic acid molecules that is derived from at least three different strains of *Staphylococcus aureus.* By increasing the number of *Staphylococcus aureus* strains to generate the plurality of nucleic acids in the array, the array more and more mimics the complete genetic potential of the *Staphylococcus aureus* species and thus the typing becomes more and more specific. Thus in a preferred embodiment the array comprises a plurality of nucleic acid molecules comprising a representation of the genomic diversity of the *Staphylococcus aureus* species. This does not mean that typing with arrays carrying a reduced number of different *Staphylococcus aureus* strains is not a valid approach; it only means that predictions and estimations become more accurate and complete.

Examples

**Example 1**

**Classification of MRSA species by (supervised) PLS-DA analysis** of **whole-genome-array differential hybridisation data.**

Fluorescently labelled genomic DNA's (gDNA) of a set of 19 different MRSA (Multiple Resistant *S. aureus*) species with known degree of epidemic character in hospitals (Epidemic or Nonepidemic) were separately hybridised to arrays coated with randomly chosen genomic DNA fragments of a mixture of 8 different *S. aureus* species (approx. 21 fragments/array, approx. 15 bp/fragment). The fluorescent hybridisation patterns were quantified resulting in a list of hybridisations signals per genomic DNA fragment for each tested species. To be more specific each array was simultaneously hybridised with 2 labelled gDNA's: one concerning a specific *S. aureus* species under investigation (labelled with Cy5), and the other concerning a standard mix of the 8 *S. aureus* species used for making of the array serving as a reference to normalise hybridisations made on all the separate slides (labelled with Cy3). The subsequent data analysis involved filtering, normalisation and cut-off-treatment of the data followed by Principal Least Square Discriminant Analysis (PLS-DA) on basis of the epidemic character of the tested *S. aureus* strains The PLS-DA analysis resulted in significant clustering of the tested *S. aureus* species according to their known epidemic character. This shows that part of the total differential hybridisation dataset contains predictive information that can be used to predict epidemicity of unknown MRSA species.

*Set of different bacterial strains*

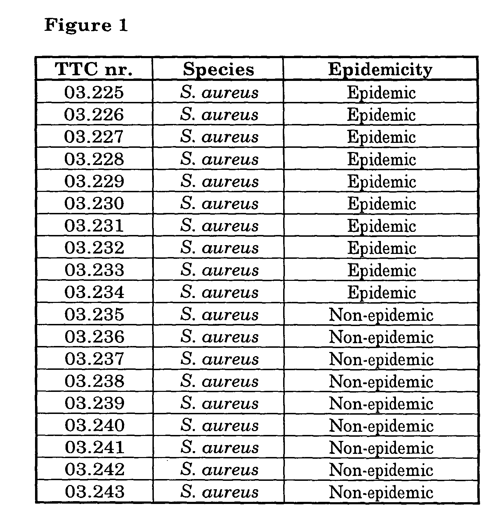

A set of 19 multiple resistant *S. aureus* strains was used for Example 1 (Fig.1). The set consisted of 19 hospital isolates. For all strains their epidemic character was abstracted from daily hospital practice (Fig.1).

*Growth and gDNA isolation of Staphylococcus strains*

*Staphylococcus* isolates were grown (via single colonies) on TSA-agar plates and/or TSA-medium (overnight, 37°) and stored as glycerol cultures (-80°). For gDNA isolation, plate grown bacteria (e.g. amount of 10-20 colonies) were resuspended in 4µl TE-buffer (10 mM Tris, 1 mM EDTA, pH7.5) in a 2 ml vial. The cells were lysed by adding 4 µl water-washed 0.1 mm Zirconium glassbeadsuspension (Biospec Products™, precooling on ice, medium-level shaking for 120 sec in a cell disrupter (minibeadbeater 8, Biospec Products™) and cooling on ice. After centrifugation (5 min, 14 krpm, 4°C), gDNA was isolated from the cleared lysate according to standard procedures (Sambrook, 1989) by extraction with phenol/chloroform/isoamylalcohol (roomtemp.), extraction with chloroform/isoamylalcohol (roomtemp.), precipitation with ethanol/Na-acetate (-20°C, spinning at 4°), washing with 70% ethanol (-20°C, spinning at 4°), drying (vacuum), dissolving the pellet in 1 µl TE-buffer with RNAseA (1-1 µg/ml) and semi-quantification of the gDNA-amount on 0.6% agarose ethidiumbromide gels (e.g. 1-5 µl preparation/spot).

*Construction of S.acureus gDNA array (slides)*

To make an array containing a genome-wide representation of the species *S. aureus*, a mixed-genomic library of the organism was made by mixing gDNA of 8 *S. aureus* strains. Strains were selected that: (a) showed each a different profile of resistance to a broad set of antibiotics (together covering most types of antibiotic-resistance), and (b) did not contain a significant plasmid band in the agarose-gel analysis of their isolated gDNA. The gDNA mix was sheared by sonication (Branson sonifier 450) and runned in several lanes of a 0.8% agarose gel. DNA-fragments (approx. 1-3 kb) were excised and isolated via binding to glass-milk (Bio101-kit). The isolated fragments were pretreatment with DNA-terminator End-repair kit (Lucigen Corp.) to facilitate efficient (blunt)cloning into bacterial plasmids (pSmartHCkan vector, CloneSmart Blunt Cloning Kit, Lucigen). Part of the ligationmix (1µl) was transformed to 25 µl *E.coli* cells (*E. cloni* 10G supreme electrocompetente cells, Lucigen) by electroporation (0,1cm-gap cuvettes Eurogentec, BioRad Pulsor, 25 uF, 2 ohms, 1,6 kV) and regeneration in TB-culture medium and plating on TY-plates with 30 µg/ml kanamycin grown overnight at 37°C. Using tooth-picks, colonies were transferred to into 96-well microtiterplates (32 plates, 150 µl/well TY medium with 30 µg/ml kanamycin). After overnight growing at 37°C, glycerol was added (final conc. 15%) and the glycerol-stocks were stored at -80°C. The genomic inserts from each clone in the well-plates were multiplied using PCR-amplification in 96-well PCR-plates (22 plates). PCR reactions contained 50 µl reactionmix/well with 1xSuperTaq buffer, 0.2mM of each dNTP (Roche Diagnostics), 0.4 µM primer L1(5'-cag tcc agt tac gct gga gtc-3') and 0.4 µM primer L1(5'-ctt tct gct atg gag gtc agg tat g-3'), 1.5 U SuperTaq-DNA-polymerase and µl glycerolstock from corresponding well of gDNA-bank. Both primers contain a free NH2-group coupled via a C6-linker to the 5' end of the primer. The following PCR-program was used: 4 min 94°C, 30x (30 sec 94°C, 30 sec 50°C, 3 min 72°C), 10 min 72°C and soaking at 4°C. Following the amplifications, the 50 µl PCR-products were transferred to 96-well roundbottom plates and precipitated by adding 150 µl NaAc/isopropanol mix (0.2M NaAc, 67% isopropanol final conc. each), incubation 1hr -80°C, spinning (1 hr, 2.5 krpm, 4°C), removal of supernatant and washing with 1 µl 70% ethanol. DNA-pellets were resuspended in 50 µl water/well, transferred to 384-well plates, dried (speed vac) and resuspended in 10 µl 3xSSC-buffer per well. The 6 resulting 384-well plates, containing approx. 21 PCR-products were used for spotting the micro-arrays. The PCR-products were spotted on series of maximal 75 "aldehyde" coated slides (Cell Associates)) using an ESI micro-array printer in combination with 24 TeleChem Stealth micro spotting quill-pins (approx 100 µm diameter). After spotting, slide surfaces were blocked by treatment at room temperature with boro-anhydride: 2x 5 min in 0.2% SDS, 2x 5min in water, 10 min in boro-anhydride buffer (1.7 g NaBH4 in 510 ml PBS-buffer and 170 ml 1% ethanol), 3x 5 min in 0.2% SDS, 3x 5 min in water, 2 sec in 1°C water, dry with $N_2$ flow. PBS (phosphate buffered saline) is 6.75 mM Na2HPO4, 1.5 mM K2HP04, 140 mM NaCl, 2.7 mM KCl pH 7.0. (1.2 g Na2HPO4, 0.2 g K2HPO4, 8.0 g NaCl, 0.2 g KCl per liter, pH 7.0).

*Labeling of gDNA*

Fluorescent labeling of gDNA was performed on 0.5-2 µg of isolated Staphylococcus gDNA for 1.5 hr at 37° in a 25 µl reaction based on BioPrime[R] DNA Labeling System (Invitrogen, Cat. No.: 18094-011). The reaction contained (final conc): 1x RandomPrimer solution (50mM Tris-HCl PH 6.8, 5mM MgC12, 30µg/ml random octamers, Bioprime[R]), 1x lowT dNTP-mixture (0.25mM dATP, 0.25mM dGTP, 0.25mM dCTP, 0.1mM dTTP), 0.06mM Cy-dUTP (Cy = either Cy5 or Cy3, 1µl of 1mM stock, Amersham Biosciences) and 20 Units DNA-polymerase (Klenow fragment; 0.5 µl of 40U/µl stock, Bioprime[R], Invitrogen). After the reaction, salts, unincorporated (labeled) nucleotides and primers were removed by purification over an Autoseq G50 column (Amersham Biosciences). After purification 1/10th part of the labeled material was used for spectrophotometric analysis to determine the quantity of DNA (A260[nm]) and Cy5 (A649[nm]) or Cy3 (A550[nm]). The remainder of the labelled material was used for array hybridisation.

*(Pre-)Hybridisation of arrays*

In preparation for hybridisation, slides were laid in Petri dishes, in 20 ml prehybridisation solution (1% BSA,, 5x SSC, 0.1% SDS, filtered through a 0.45 µm filter, 42°) and were gently shaken (by mild rotation) for 45 min at 42°.
Next the slide was washed 2x in 40 ml water (in a 40 ml capped tube) and quickly dried using an $N_2$-gun.
The appropriate gDNA samples that were labeled with Cy5-dUTP and Cy3-dUTP were combined with 4 µl yeast tRNA (25 µg/µl), dried (using a SpeedVac), re-dissolved in 40 µl EasyHyb solution (Roche Applied Science), denatured (1.5 min, 95°C), spinned down briefly (1 sec, 10 krpm), pipetted on a pre-warmed (42°C metal plate) dry prehybridised array, covered with a plastic cover slip (Hybrislip, Molecular Probes), inserted in a water-vapour-saturated prewarmed (42°C) hybridisation chamber (Corning) and hybridised overnight in a 42°C waterbath.
After hybridisation the arrays were washed by shaking slides 4x in 40ml of (different) buffers in capped 40 ml tubes (wash-buffer1: 1x SSC, 0.2% SDS, 37°, 5-10 sec; wash-buffer2: 0.5x SSC, 37°C, 5-10 sec; wash-buffer 3 and 4: 0.2x SSC, 20°C, each 10 min).

*Scanning and image analysis*

After washing, slides were stored in the dark (to prevent decay of Cy-fluorescence) or directly used for scanning the fluorescent Cy dyes with a scanning device (ScanArray 4000 from Perkin Elmer). A quickscan (resolution 30 µm/pixel) was performed to select optimal laser- (intensity) and detection (photomultiplier) settings in order to prevent excess of low signals or saturated signals. Slides were scanned twice: for Cy5- and Cy3-fluorescence. Digital scans were quantified with ImaGene software (BioDiscovery Inc., version 4.2) resulting in a spot identity, and Signal (S) and Background (B) values for both Cy5 and Cy3, for each spot on the array. The data were stored in electronic files and used for further data processing.

*Data pre-processing*

By using spreadsheet software (Excel, Microsoft) the following calculations were made for each spot: S-B values for Cy5 and Cy3, Cy5/Cy3 ratio's [R=Cy5(S-B)]/[(Cy3(S-B)]. Low quality data were removed (e.g. spots having Cy3 data with S<2B). Then, a normalisation factor N was calculated for each slide, based on average Cy5- and Cy3-signal for all spots on a slide (N = [averageCy5(S-B)]/[averageCy3(S-B)]. Next, normalised ratio's ($R_n$) were calculated for each spot ($R_n$ = R/N) on all arrays. A matrix (= dataset) of normalised ratio's per spot for many slides (slides relate with *Staphylococcus* strains) was used for further data preprocessing.

Since the Cy3 signal is generally present for most spots (Cy3-labeled reference gDNA pool of 8 strains was hybridised to all slides), and the Cy5 signal can vary (Cy5-labeled gDNA of different strains were each hybridised to single slides), the Cy5/Cy3 ratio can in theory have two values (1 or 0) if a gDNA fragment is present or absent respectively, in the Cy5 tested strain. In practice, however, these values vary around 1 and 0. Therefore, in many analyses cut-off values for 0 and 1 were applied on the ratio-dataset before further analysis (e.g. $R_n<0.5$ and $R_n>0.5$ were replaced by 0 and 1 respectively, or, $R_n<0.3$ and $R_n>0.7$ were replaced by 0 and 1 respectively while keeping $R_n$ values between 0.3 and 0.5). These "cut-off datasets" were used for the final data analysis.

*PLS-DA data analysis*

A "cut-off dataset" was analysed by Principal Least Square Discriminant Analysis (PLS-DA) on basis of the known epidemic character of the tested MRSA strains.
Comparable results can be obtained with no or alternative scaling methods (e.g. autoscaling) or alternative multivariate methods.

*Results*

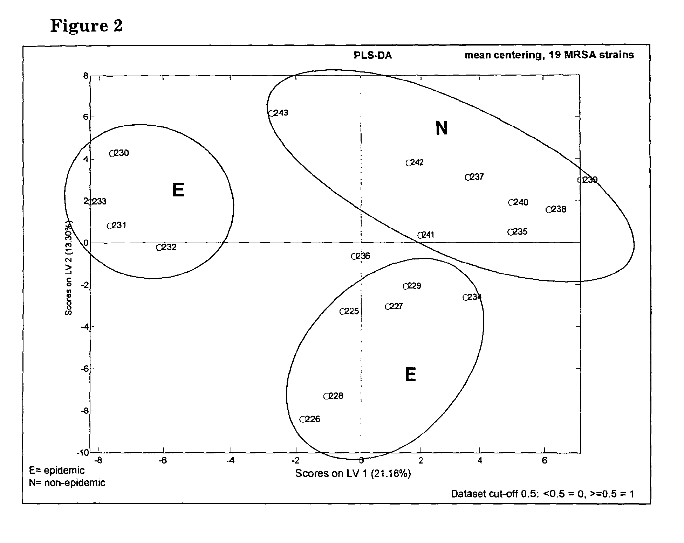

A set of 19 different S. *aureus* isolates was processed for PLS-DA analysis of data from differential hybridisation on whole-genome microarrays (Fig.2).

The PLS-DA analyses resulted in significant clustering of the MRSA species according to their known epidemic character (Fig.2, E=Epidemic, N=Non-epidemic).
This shows that part of the total differential hybridisation dataset contains predictive information that can be used to predict epidemicity of unknown MRSA species.

**Brief description of the drawings**

**Figure 1**

**List of MRSA isolates, id's and epidemic character.**

Each MRSA strain is identified by a unique TNO Type Collection number (TTC nr, 1st column). Each strain was characterised as *S. aureus* species by Ribroprint classification (2nd column). Epidemic character was determined from daily hospital practice (3rd column).

**Figure 2**

**Classification for epidemicity of MRSA species by (supervised) PLS-DA analysis of whole-genome-array differential hybridisation data.**

Cy-labelled genomic DNA of 19 different MRSA strains was hybridised to arrays containing a representation of the *S. aureus* genome. The quantified fluorescent hybridisation patterns of the *Staphylococcus* species, representing a highly complex n-dimensional data-set, were analysed with Partial-Least-Square-Discrimminant-Analysis (PLS-DA) on basis of the known epidemic character of each MRSA strain. The PLS-DA plot below shows single point projections of each single-species complex hybridisation pattern in a 2-dimensional plane (small circles, with text indicating strain TTC.03 number mentioned in **Fig.1)**. In duplo hybridised strains are indicated with bold text. Based on a specific part of the dataset, the PLS-DA analysis is able to cluster the species in two separate clusters (manually placed ellipses, indicated E=Epidemic and N=non-epidemic) according to their known epidemicity. Note: Cy5/Cy3-ratio's were transformed to a 0 and 1 dataset by cut-off 0.5. PLS-DA-scaling was Mean centering. The non-epidemic strain "236" was PLS-DA classified intermediate the E- and N -cluster.

**Figure 3**

Model complexity

**Figure 4**

Illustration of discriminant analysis. D is the discriminant axis, P is a projection line, $X_1$ and $X_2$ are two variables and x and o represent samples from two different groups.

**Claims**

1. A method for typing sample nucleic acid derived or obtained from a *Staphylococcus aureus* strain comprising, providing an array comprising a plurality of nucleic acid molecules obtained or derived from *Staphylococcus aureus* wherein said array nucleic acid molecules comprise an average size of between about 200 to 5000 nucleotides and hybridising said array with *Staphylococcus aureus* sample nucleic acid, wherein said sample nucleic acid comprises an average size of between about 50 to 5000 nucleotides and comparing the sample hybridisation pattern with a reference pattern.

2. A method according to claim 1, wherein said plurality of nucleic acid molecules is derived from at least two different strains of *Staphylococcus aureus.*

3. A method according to claim 1 or claim 2, wherein said sample nucleic acid is derived from a pure culture of *Staphylococcus aureus.*

4. A method according to any one of claims 1-3, further comprising comparing the sample pattern with at least one other reference pattern.

5. A method according to claim 4, comprising comparing the hybridisation pattern with at least 2, preferably at least 5 and more preferably at least 50 reference patterns.

6. A method according to claim 4 or claim 5, wherein said comparison comprises Partial Least Square-Discriminant Analysis (PLS-DA) of the reference patterns together with the pattern generated with the sample nucleic acid and wherein at least one phenotypic parameter of which the values are known for the reference patterns, (and which information wherein this parameter is used to supervise the PLS-DA analysis), is typed for the sample nucleic acid or the natural source it is derived from.

7. A method according to claim 6, further comprising clustering patterns based on the supervised PLS-DA analysis.

8. A method according to claim 7, further comprising typing said sample nucleic acid on the basis of the presence or absence in a cluster.

9. A method according to any one of claims 1-8, wherein at least one of said reference patterns is a pattern generated by nucleic acid of nucleic acid from a *Staphylococcus aureus* strain.

10. A method according to any one of claims 4-9, wherein essentially all reference patterns are generated by nucleic acid of *Staphylococcus aureus* strains.

11. A method according to any one of claims 1-10, wherein said typing comprises determining whether the *Staphylococcus aureus* said sample nucleic acid is derived from is an epidemic strain.

12. An array comprising a plurality of nucleic acid molecules derived or obtained from *Staphylococcus aureus* wherein said nucleic acid molecules comprise an average size of between about 200 to 5000 nucleotides.

13. An array according to claim 12, wherein said plurality of nucleic acid molecules are derived from a natural source.

14. An array according to claim 13, wherein said plurality of nucleic acid molecules is derived from at least two different strains of *Staphylococcus aureus.*

15. An array according to claim 17, wherein said plurality of nucleic acid molecules comprises at least one genome of a *Staphylococcus aureus* strain.

16. An array according to claim 15, wherein said plurality of nucleic acid molecules comprises a representation of the genomic diversity of the *Staphylococcus aureus* species.

17. Use of an array according to any one of claims 12-16, for determining whether a *Staphylococcus aureus* strain in a sample is an epidemic strain.

**Figure 1**

| TTC nr. | Species | Epidemicity |
|---------|---------|-------------|
| 03.225 | *S. aureus* | Epidemic |
| 03.226 | *S. aureus* | Epidemic |
| 03.227 | *S. aureus* | Epidemic |
| 03.228 | *S. aureus* | Epidemic |
| 03.229 | *S. aureus* | Epidemic |
| 03.230 | *S. aureus* | Epidemic |
| 03.231 | *S. aureus* | Epidemic |
| 03.232 | *S. aureus* | Epidemic |
| 03.233 | *S. aureus* | Epidemic |
| 03.234 | *S. aureus* | Epidemic |
| 03.235 | *S. aureus* | Non-epidemic |
| 03.236 | *S. aureus* | Non-epidemic |
| 03.237 | *S. aureus* | Non-epidemic |
| 03.238 | *S. aureus* | Non-epidemic |
| 03.239 | *S. aureus* | Non-epidemic |
| 03.240 | *S. aureus* | Non-epidemic |
| 03.241 | *S. aureus* | Non-epidemic |
| 03.242 | *S. aureus* | Non-epidemic |
| 03.243 | *S. aureus* | Non-epidemic |

## Figure 2

Figure 3

Figure 4

**European Patent Office**

## EUROPEAN SEARCH REPORT

Application Number

EP 04 07 6394

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 2003/198943 A1 (DUFOUR SOPHIE ET AL) 23 October 2003 (2003-10-23) see whole doc. esp. claims and expl. 27, p.22 ----- | 1-17 | C12Q1/68 |
| X | HAMELS S ET AL: "CONSENSUS PCR AND MICROARRAY FOR DIAGNOSIS OF THE GENUS STAPHYLOCOCCUS, SPECIES, AND METHICILLIN RESISTANCE" BIOTECHNIQUES, EATON PUBLISHING, NATICK, US, vol. 31, no. 6, December 2001 (2001-12), pages 1364-1366,1368, XP001153876 ISSN: 0736-6205 see whole doc. esp. material and methods ----- | 1-17 | |
| X | LEEUWEN VAN W B ET AL: "Multilocus sequence typing of Staphylococcus aureus with DNA array technology" JOURNAL OF CLINICAL MICROBIOLOGY, WASHINGTON, DC, US, vol. 41, no. 7, July 2003 (2003-07), pages 3323-3326, XP002280375 ISSN: 0095-1137 see whole doc. esp. p.3325, 1.col., 2. par. ----- | 1-17 | |
| X | US 6 228 575 B1 (GINGERAS THOMAS R ET AL) 8 May 2001 (2001-05-08) see whole doc. esp. claims (e.g. cl.77) ----- -/-- | 1-17 | |

TECHNICAL FIELDS SEARCHED (Int.Cl.7)

C12Q

The present search report has been drawn up for all claims

| Place of search | Date of completion of the search | Examiner |
|---|---|---|
| Munich | 29 September 2004 | Mueller, F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**European Patent Office**

## EUROPEAN SEARCH REPORT

Application Number

EP 04 07 6394

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| Y | CHO J-C ET AL: "Bacterial species determination from DNA-DNA hybridization by using genome fragments and DNA microarrays" APPLIED AND ENVIRONMENTAL MICROBIOLOGY, WASHINGTON,DC, US, vol. 67, no. 8, August 2001 (2001-08), pages 3677-3682, XP002953482 ISSN: 0099-2240 see whole doc. esp. p.3692, 1.col., last par. ----- | 1-17 | |
| Y | KURODA M ET AL: "Whole genome sequencing of meticillin-resistant Staphylococcus aureus" LANCET THE, LANCET LIMITED. LONDON, GB, vol. 357, no. 9264, 21 April 2001 (2001-04-21), pages 1225-1240, XP004246103 ISSN: 0140-6736 * the whole document * ----- | 1-17 | |
| A | TROESCH ET AL: "MYCOBACTERIUM SPECIES IDENTIFICATION AND RIFAMPICIN RESISTANCE TESTING WITH HIGH-DENSITY DNA PROBE ARRAYS" JOURNAL OF CLINICAL MICROBIOLOGY, WASHINGTON, DC, US, vol. 37, no. 1, January 1999 (1999-01), pages 49-55, XP002130858 ISSN: 0095-1137 * the whole document * ----- | | TECHNICAL FIELDS SEARCHED (Int.Cl.7) |

The present search report has been drawn up for all claims

| Place of search | Date of completion of the search | Examiner |
|---|---|---|
| Munich | 29 September 2004 | Mueller, F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.......................................................................................
& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 04 07 6394

This annex lists the patent family members relating to the patent documents cited in the above-mentioned European search report.
The members are as contained in the European Patent Office EDP file on
The European Patent Office is in no way liable for these particulars which are merely given for the purpose of information.

29-09-2004

| Patent document cited in search report | | Publication date | Patent family member(s) | | Publication date |
|---|---|---|---|---|---|
| US 2003198943 | A1 | 23-10-2003 | EP | 1136566 A1 | 26-09-2001 |
| | | | AU | 4212401 A | 23-10-2001 |
| | | | WO | 0177372 A2 | 18-10-2001 |
| | | | EP | 1266034 A2 | 18-12-2002 |
| | | | JP | 2003530116 T | 14-10-2003 |
| | | | US | 2002106646 A1 | 08-08-2002 |
| US 6228575 | B1 | 08-05-2001 | AU | 2189397 A | 28-08-1997 |
| | | | EP | 0937159 A1 | 25-08-1999 |
| | | | JP | 2000504575 T | 18-04-2000 |
| | | | WO | 9729212 A1 | 14-08-1997 |

EPO FORM P0459

For more details about this annex : see Official Journal of the European Patent Office, No. 12/82